(19) Europäisches Patentamt

European Patent Office

Office européen des brevets

(11) **EP 0 688 769 B1**

(12) **FASCICULE DE BREVET EUROPEEN**

(45) Date de publication et mention
de la délivrance du brevet:
**22.03.2000 Bulletin 2000/12**

(51) Int Cl.⁷: **C07D 307/83**

(21) Numéro de dépôt: **95401385.0**

(22) Date de dépôt: **14.06.1995**

(54) **Méthode de préparation de la 2-coumaranone**

Verfahren zur Herstellung von 2-Coumaranon

Method of preparation of the 2-coumaranone

(84) Etats contractants désignés:
**AT BE CH DE ES FR GB IT LI LU NL SE**

(30) Priorité: **22.06.1994 FR 9407651**

(43) Date de publication de la demande:
**27.12.1995 Bulletin 1995/52**

(73) Titulaire: **Clariant (France) S.A.**
**92800 Puteaux (FR)**

(72) Inventeurs:
 • **Vallejos, Jean-Claude**
 **F-75018 Paris (FR)**
 • **Perrard, Alain**
 **F-69110 Ste Foy les Lyon (FR)**
 • **Christidis, Yani**
 **F-75019 Paris (FR)**
 • **Gallezot, Pierre**
 **F-69001 Lyon (FR)**

(74) Mandataire: **Rinuy, Santarelli**
**14, avenue de la Grande Armée**
**75017 Paris (FR)**

(56) Documents cités:
 **EP-A- 0 041 656** **FR-A- 2 686 880**

 • **JOURNAL OF ORGANIC CHEMISTRY, vol. 47,
 no. 12, 4 Juin 1982 EASTON US, pages
 2491-2493, T. FUKAGAWA ET AL.
 'Palladium-promoted intramolecular aromatic
 nuclear acyloxydation: preparation of
 2-coumaranone.'**
 • **CHEMISCHE BERICHTE, vol. 96, 1963
 WEINHEIM DE, pages 826-839, A. MONDON ET
 AL. 'Neue abkömmlinge der
 Cyclohexanon-(1)-essigsäure-(2)'**

## Description

La présente invention a pour objet un procédé de préparation de la 2-coumaranone, désignée également 3H-benzofuranone-2.

F. Fukagawa et al décrivent, dans J. Org. Chem. Vol. 47, N° 12, 1982, P 2491-93, l'obtention de la 2-coumaranone par cyclisation de l'acide phénylacétique en présence du système $Pd(OAc)_2$-$K_2S_2O_8$-$CH_3SO_3H$.

FR-A-2 686 880 décrit un procédé de préparation de la chloro-5,3H-benzofuranone-2 par réaction de l'acide glyoxylique avec du parachlorophénol en présence d'acide phosphonique et de quantités catalytiques d'iode ou d'acide iodhydrique.

EP-A-0 041 656 décrit la préparation d'un mélange de 2-éthoxy-4-oxo-2,3,4,5,6,7-hexahydrocoumarone et de 2-(2',2'-diéthoxyétyhyl)-1,3-cyclohexanedione par couplage électrooxydatif de la 1,3-cyclohexanedione avec l'éthylvinyléther en présence d'éthanol.

La 2-coumaranone est un produit connu, largement décrit dans la littérature (cf Beil. 17, 309 ; I, 159 ; II, 331). C'est la lactone de l'acide orthohydroxyphénylacétique et elle est utilisée comme matière première en synthèse organique pour accéder à divers produits destinés à l'agriculture ou présentant des effets physiologiques. De ce fait, on recherche en permanence des procédés permettant de l'obtenir rapidement et à bon marché à partir de produits commerciaux peu onéreux. Or, la demanderesse a découvert avec étonnement un procédé de préparation rapide de la 2-coumaranone à partir d'acide glyoxylique et de cyclohexanone.

La présente invention a donc pour objet un procédé de préparation de la 2-coumaranone caractérisé par le fait que l'on fait réagir, en phase vapeur, de l'acide glyoxylique avec de la cyclohexanone, en présence d'un catalyseur de déshydrogénation.

Par l'expression "catalyseur de déshydrogénation", on désigne un catalyseur connu pour l'aromatisation des cycles à 6 chaînons comme ceux cités dans Advanced Organic Chemistry, Jerry March, 3ème édition, pages 1052-1054, J.Willey, Interscience, New-York, 1985, ainsi que dans Houben-Weyl, Phenole, Tome 2, pages 701-716, Georg Thieme - Stuttgart, 1976. Préférentiellement le catalyseur de déshydrogénation est choisi dans le groupe constitué par le palladium et le platine.

Dans des conditions préférentielles de mise en oeuvre de l'invention, le procédé ci-dessus décrit est réalisé de la manière suivante :

- à une température supérieure ou égale à 150°C,
- en utilisant un gaz vecteur constitué par un mélange de 0 à 100 % de diazote, N2, et de 100 à 0 % de dihydrogène, $H_2$.
- en dissolvant l'acide glyoxylique et la cyclohexanone dans un solvant ou un mélange de solvants présentant un point d'ébullition inférieur à 150°C, de préférence dans de l'acide acétique ou un mélange eau-acide acétique.
- en mettant en oeuvre des quantités environ stoechiométriques d'acide glyoxylique et de cyclohexanone, ou un léger excès de cyclohexanone,
- en utilisant un catalyseur à base de platine ou de palladium déposé sur un support solide inerte tel que l'alumine $\alpha$, l'alumine $\gamma$, la silice, le carbure de silicium, le charbon, présentant une surface spécifique supérieure à 1 $m^2$ par gramme et préférentiellement supérieure à 50 $m^2$ par gramme, à une concentration supérieure ou égale à 0,5 % en poids.

Dans des conditions plus préférentielles, le procédé ci-dessus décrit est mis en oeuvre en faisant passer sur un lit catalytique constitué par du palladium métallique déposé sur du charbon, à une température supérieure à 200°C, un mélange gazeux issu de l'évaporation d'une solution dans l'acide acétique contenant des quantités équimoléculaires de cyclohexanone et d'acide glyoxylique en solution aqueuse dans un gaz vecteur constitué de diazote et de dihydrogène dans des proportions variables.

Selon une variante de l'invention, le procédé ci-dessus décrit est réalisé en deux stades : dans un premier stade, on fait réagir selon un procédé connu, l'acide glyoxylique avec de la cyclohexanone pour obtenir un mélange en proportions variables d'acide (oxo-2 cyclohexylidène)acétique sous ses différentes formes stéréoisomériques et/ou sous formes de diverses $\gamma$ lactones internes, puis, dans un deuxième stade, on prépare la 2-coumaranone en faisant passer, à l'état de vapeur, le mélange réactionnel brut obtenu au premier stade sur un catalyseur de déshydrogénation identique à celui utilisé précédemment.

L'invention a donc également pour objet un procédé de préparation de la 2-coumaranone caractérisé par le fait que l'on fait passer, à l'état de vapeur, sur un catalyseur de déshydrogénation, identique à celui défini précédemment, le brut réactionnel obtenu par la condensation, réalisée selon un procédé connu en soi, de l'acide glyoxylique avec de la cyclohexanone.

Dans des conditions préférentielles de mise en oeuvre de la variante de l'invention, le procédé ci-dessus décrit est réalisé de la manière suivante :

- La condensation de l'acide glyoxylique avec la cyclohexanone est réalisée
- à une température supérieure à 50°C,
- en solution dans un mélange constitué par 0 à 100 % d'eau et de 100 à 0% d'acide acétique,
- avec des quantités stoechiométriques de réactifs et une concentration comprise entre 10 et 50 % en poids,
- le mélange réactionnel brut de la condensation de l'acide glyoxylique avec de la cyclohexanone est vaporisé à une température supérieure ou égale à 150°C, puis il est entraîné sur le catalyseur à l'aide d'un gaz vecteur constitué par un mélange de 100 à 0% de diazote et de 0 à 100 % de dihydrogène.
- le catalyseur est à base de platine ou de palladium déposé sur un support solide inerte tel que l'alumine $\alpha$, l'alumine $\gamma$,, la silice, le carbure de silicium, le charbon, présentant une surface spécifique supérieure à 1 $m^2$ par gramme et préférentiellement supérieure à 50 $m^2$ par gramme, à une concentration supérieure ou égale à 0,5 %.

La 2-coumaranone est isolée du mélange gazeux par des moyens connus en soi. Préférentiellement, le mélange gazeux est refroidi à la température ambiante, puis la 2-coumaranone formée est isolée par distillation, les produits de départ non transformés sont recyclés ainsi que le ou les solvants.

Au cours de la réaction de déshydrogénation de l'acide (oxo-2 cyclohexylidène)acétique en 2-coumaranone, dans certaines conditions opératoires, on observe la formation, comme produits secondaires de la réaction, en quantités non négligeables, d'acide cyclohexanone-2 acétique et d'acide cyclohexanol-2 acétique.

Ces produits peuvent également être préparés par d'autres voies de synthèse : Melvin S.N et al J. Am. Chem. Soc.,p 233, feb. 1945 ; Mondon A et al, Chem. Ber., 96, 826 (1963).

L'acide cyclohexanone-2 acétique et l'acide cyclohexanol-2 acétique conduisent également à la formation de 2-coumaranone si on leur fait subir le procédé de déshydrogénation décrit ci-dessus.

L'invention a donc encore pour objet un procédé de préparation de la 2-coumaranone caractérisé en ce que l'on fait passer, en phase vapeur, de l'acide cyclohexanone-2 acétique ou de l'acide cyclohexanol-2 acétique sur un catalyseur de déshydrogénation.

Cette propriété est particulièrement intéressante puisqu'elle permet de recueillir les produits secondaires de la réaction de transformation de l'acide (oxo-2 cyclohexylidène) acétique en 2-coumaranone, pour les recycler comme réactifs de départ dans ce même procédé.

La condensation de l'acide glyoxylique avec la cyclohexanone pour obtenir l'acide (oxo-2 cyclohexylidène) acétique sous ses différentes formes stéréoisomériques et/ou lactoniques ainsi que les différents produits obtenus sont notamment décrits dans DE-A-1 955 375 et par K.W Rosenmund et al, Arch.Pharm., 287, 441, (1954) ; A.Mondon et al, Chem. Ber., 96, 826, (1963) ; A.W Noltes et al, Rec. Trav. Chim., 80, 1334 (1961) ; Y.Arbuzov et al, Zhur.Obschei Khim., 32, 3676 (1962) ; M.N KOLOSOV et al, Zhur.Obschei Khim., 32, 2983, (1962) ; F.BONADIES et al, Gazz. Chim., Ital., 113, 421 (1983).

Les exemples suivants illustrent l'invention sans toutefois la limiter.

## EXEMPLE 1

On chauffe 24 heures au reflux un solution contenant :

- 736 g (7,5 moles) de cyclohexanone,
- 694 g d'acide glyoxylique à 80 % en poids dans l'eau, soit 7,5 moles d'acide glyoxylique à 100 %,
- 3520 g d'acide acétique pur,

puis la solution obtenue est concentrée sous pression réduite jusqu'à l'obtention d'un poids de 3520 g. A ce stade, cette solution, désignée S1, contient :

- 3,2 % de cyclohexanone non transformée,
- 9 % d'acide (oxo-2 cyclohexylidène) acétique, isomère trans, désigné 1, décrit par M.N Kolosov et al,
- 17 % de la lactone de l'acide (dihydroxy-2,2 cyclohexylidène) acétique, désignée 2, décrite notamment par Yu Wang et al, Hua Hsueh Hsueh Pao, 26 (2), 84 (1960), et 28 (6) 351 (1962).
- 1,3 % de la lactone de l'acide (hydroxy-2 cyclohexène-2 ylidène) acétique, désignée 3, décrite par Yu Wang et al,
- 69,5 % d'acide acétique.

Ces déterminations ont été effectuées par chromatographie en phase liquide haute performance et par dosage acidimétrique.

## EXEMPLE 2

On chauffe 90 minutes à 130°C, sous pression dans un réacteur fermé, une solution contenant :

- 245 g (2,5 moles) de cyclohexanone
- 296 g d'une solution aqueuse commerciale d'acide glyoxylique à 50 % en poids, soit 2 moles d'acide glyoxylique,
- 228 g d'acide acétique pur,

puis le milieu réactionnel est refroidi à la température ambiante. On obtient ainsi une solution, désignée S2, contenant pondéralement :

- 9 % de cyclohexanone,
- 15 % de 1,
- 19 % de 2,
- 57 % d'un mélange acide acétique-eau.

## EXEMPLES 3-9

Dans un vaporisateur maintenu à une température de 200°C, on vaporise la solution SI, avec débit de 6ml/h et les vapeurs sont entraînées par un courant de diazote avec un débit de 200 Nl/h (1 Nl = 22,4 1) vers le réacteur maintenu à une température de 250°C.

Le réacteur est un cylindre de diamètre intérieur de 26 mm et de longueur 200 mm, vertical, bouché à son extrémité inférieure par un verre fritté, de volume utile de 80 ml, rempli par des billes de verre, de diamètre 2 mm, de la manière suivante : 15 ml de billes de verre, puis M g d'un catalyseur Ca dilué avec 20 ml de billes de verre et enfin 40 ml de billes de verre. La masse M de catalyseur Ca correspond à une masse de métal actif constante de 0,05 g. A la sortie du réacteur, les gaz chauds sont refroidis et condensés par passage dans des pièges immergés dans de l'azote liquide. Les produits condensés dans les pièges sont réunis puis analysés par chromatographie en phase liquide à haute performance (CLHP). Les résultats obtenus après deux heures de fonctionnement, en fonction du catalyseur, sont mentionnés dans le tableau A dans lequel TTG signifie "taux de transformation globale" et SC la "sélectivité de la réaction" pour la 2-coumaranone.

$$TTG = \frac{\Sigma m \text{ (produits formés)}}{\Sigma m \text{ (réactifs résiduels)} + \Sigma m \text{ (produits formés)}}$$

$$SC = \frac{m \text{ (2-coumaranone)}}{\Sigma m \text{ (produits formés)}}$$

Σm désigne une somme de masses
m désigne la masse d'un produit

## EXEMPLE 10

On procède comme dans les exemples 3 à 9 en utilisant $S_2$ comme produit de départ, que l'on injecte à un débit de 12 ml/h, avec comme catalyseur, une quantité totale de 250 mg de palladium déposé à 2,5 % sur l'alumine $\alpha$ (catalyseur G). Les produits de la réaction sont piégés dans de l'acétonitrile pendant une heure.

TABLEAU A

| Catalyseur Ca | TTG (%) | SC (%) |
|---|---|---|
| **A** - 2,5 % Pd/C, préparé au laboratoire | 97 | 91 |
| **B** - 1 % Pd/C, provenance DEGUSSA E 152 X H/D | 83 | 88 |
| **B'** - 1 % Pd/C, provenance DEGUSSA E 152 X HD/broyé | 98 | 87 |
| **C** - 0,8 % Pd/C, provenance JOHNSON MATTHEY type 86 | 78 | 91 |
| **D** - 0,8 % Pd/C, provenance ENGELHARD ES Cat 19 | 91 | 89 |
| **E** - 0,5 % Pd/C, provenance ENGELHARD Code 4586 | 23 | 65 |

TABLEAU A   (suite)

| Catalyseur Ca | TTG (%) | SC (%) |
|---|---|---|
| **F** - 0,5 Pt/Al$_2$O$_3$, alumine $\gamma$, provenance ENGELHARD code 4751 | 29 | 83 |
| **G** - 2,5 % Pd/Al$_2$O$_3$, alumine $\alpha$ préparé au laboratoire | 70 | 80 |

Le catalyseur A préparé au laboratoire a été obtenu de la manière suivante :

100 g de charbon NORIT ROX 0,8 sous forme de granulés (1 = 3 mm, diamètre 0,8 mm) sont traités vers 0°C avec 825 ml d'une solution aqueuse d'hypochlorite de sodium titrée à 4,8 % de chlore actif, puis le solide est successivement lavé avec de l'acide chlorhydrique 1,25 N et de l'eau avant d'être séché sous pression réduite à 100°C. Dans 600 ml d'ammoniaque 1N, on introduit 38 g de charbon oxydé obtenu précédemment, puis dans cette suspension on introduit sous agitation, 5 g de chlorure de tétraamine de palladium dissous dans 40 ml d'eau. Après 16 heures d'agitation, le solide est isolé, lavé à l'eau, séché à 100°C en atmosphère inerte, puis il est progressivement chauffé à 200°C sous un courant d'argon et enfin il est traité par un courant de dihydrogène (250 ml/min) de 20 à 300°C (1°C/min) puis durant 3 heures à 300°C avant d'être refroidi à 20°C sous dihydrogène. Enfin, il est traité par un courant de diazote (50ml/mn) contenant 1 % de dioxygène pendant 2 heures. On obtient ainsi un catalyseur contenant 2,5 % en poids de palladium.

Le catalyseur G préparé au laboratoire a été obtenu de la manière suivante :

Dans un bêcher, 9,527 g d'alumine $\alpha$, de surface spécifique 10m$^2$/g, commercialisé par la Société Degussa sous le nom de support N° 272, sont imprégnés d'une solution acide de tétrachloropalladate de sodium, soit 0,7013 g de tétrachloropalladate de sodium dissous dans 1 ml d'acide chlorhydrique à 36 %, auquel on additionne la quantité d'eau nécessaire pour recouvrir les grains d'alumine.

Le mélange est séché à l'air libre à une température de 50°C pendant 16 heures, puis sous pression réduite à 100°C pendant huit heures.

Le catalyseur obtenu est réduit par un courant d'hydrogène diffusé à un débit de 15 l/h tandis que l'on élève la température depuis la température ambiante jusqu'à 200°C à raison de 1°C/min.

On laisse revenir le catalyseur à température ambiante sous un courant d'hydrogène puis on le met sous argon.

### EXEMPLES 11-12

Dans le même appareillage que celui utilisé dans les exemples 3-9, on effectue au départ de la solution S2 et en utilisant 5 g du catalyseur **B** à 1% de palladium déposé sur charbon commercialisé par la Société DEGUSSA sous la référence E 152 X H/D. Le vaporisateur est maintenu à une température de 200°C, le débit de la solution S2 est de 6ml/h et le débit de gaz vecteur est de 200 Nl/h. Les autres conditions opératoires ainsi que les résultats obtenus sont mentionnés dans le tableau B.

TABLEAU B

| EXEMPLES | 11 | 12 |
|---|---|---|
| Gaz vecteur : - nature<br>                     - composition (%) | H$_2$/N$_2$<br>6/94 | H$_2$<br>100 |
| Durée de fonctionnement (h) | 6 | 8 |
| TTG (%) | 89 | 98 |
| SC (%) | 82,5 | 58 |

### EXEMPLE 13

Dans 228 g d'acide acétique pur, on dissout 245 g (2,5 moles) de cyclohexanone et 296 g d'une solution aqueuse commerciale d'acide glyoxylique à 50 % en poids, soit 2 moles. On obtient ainsi une solution désignée S3.

Dans le même appareillage que celui utilisé dans les exemples 3-12, on effectue la réaction au départ de la solution S3, en utilisant 5 g du catalyseur **B** à 1% de palladium déposé sur charbon commercialisé par la Société DEGUSSA sous la référence E 152 X H/D.

Le vaporisateur est maintenu à une température de 200°C. Le réacteur est maintenu à une température de 300°C, le gaz vecteur est un mélange de diazote et de dihydrogène 96/4, et son débit est de 300 Nl/h. Le débit de la solution S3 est de 6 ml/h.

Après deux heures de fonctionnement, on atteint un TTG de 14 % et un SC de 20 %.

EXEMPLE 14 : Mise en évidence d'acide cyclohexanone-2 acétique.

Dans le réacteur tubulaire des exemples 3 à 13, contenant 0,05 g de catalyseur B, on introduit à un débit de 6ml/h et sous débit de 200Nl/h d'hydrogène, la solution S2, la température du vaporisateur étant de 220°C et celle du réacteur de 250°C.

Après 20 heures de fonctionnement, on piège l'aérosol dans de l'acétonitrile pendant 1 heure, en sortie de réacteur.

On mesure par analyse HPLC et CPG, le rendement en mole de chacun des produits d'arrivée.

| PRODUIT | RENDEMENT |
|---|---|
| 2-coumaranone | 29,8 % |
| orthocrésol | 2,3 % |
| acide phénylacétique | 1,9 % |
| acide cyclohexanone-2 acétique | 14,9 % |
| lactone de l'acide cyclohexanol-2 acétique | 1,3 % |

## EXEMPLE 15

Déshydrogénation de l'acide cyclohexanone-2 acétique.

On prépare une solution à 40 % en poids d'acide cyclohexanone-2 acétique dans de l'acide acétique.

Dans un réacteur tubulaire contenant 70 mg de Pd métal déposé à 0,5% sur alumine (catalyseur Engelhardt type Escat 16) on introduit à un débit de 10 ml/h et sous un courant d'azote de 200 Nl/h la solution acétique précédente.

La température du vaporisateur est de 220°C et celle du réacteur de 250°C.

Après piégeage pendant une heure de fonctionnement par barbotage dans 150 ml d'acétonitrile refroidis par un bain glace-eau, on mesure par HPLC et CPG les rendements en moles en produits formés.

| PRODUIT | RENDEMENT |
|---|---|
| 2-coumaranone | 19,1 % |
| orthocrésol | 9,6 % |
| acide phénylacétique | 2,3 % |
| lactone de l'acide cyclohexanolacétique | 6 % |
| Toluène | 4,4 % |

**Revendications**

1. Procédé de préparation de la 2-coumaranone caractérisé par le fait que l'on fait réagir, en phase vapeur, de l'acide glyoxylique avec de la cyclohexanone, en présence d'un catalyseur de déshydrogénation.

2. Procédé de préparation de la 2-coumaranone caractérisé par le fait que l'on fait passer, à l'état de vapeur, sur un catalyseur de déshydrogénation, le brut réactionnel obtenu par la condensation, réalisée selon un procédé connu en soi, de l'acide glyoxylique avec de la cyclohexanone.

3. Procédé de préparation de la 2-coumaranone, caractérisé par le fait que l'on fait passer, à l'état de vapeur, sur un catalyseur de déshydrogénation, une solution d'acide cyclohexanone-2 acétique et/ou d'acide cyclohexanol-2 acétique.

4. Procédé selon la revendication 1 ou 2, caractérisé en ce qu'il comprend une étape de recyclage des produits secondaires de la réaction, l'acide cyclohexanol-2 acétique et l'acide cyclohexanone-2 acétique, suivant le procédé de la revendication 3.

**5.** Procédé selon l'une quelconque des revendications 1 à 4, caractérisé par le fait que l'on utilise un gaz vecteur constitué par un mélange de 100 à 0% de diazote et de 0 à 100 % de dihydrogène pour entraîner le mélange réactionnel sur le catalyseur de déshydrogénation.

**6.** Procédé selon l'une quelconque des revendications 1 à 5, caractérisé par le fait que l'on opère à une température supérieure ou égale à 150°C.

**7.** Procédé selon l'une quelconque des revendications 1 à 6, caractérisé par le fait que le catalyseur de déshydrogénation est choisi dans le groupe constitué par le palladium et le platine.

**8.** Procédé selon l'une quelconque des revendications 1 à 7, caractérisé par le fait que le catalyseur de déshydrogénation est déposé sur un support solide inerte présentant une surface spécifique supérieure à 1 $m^2$ par gramme.

**9.** Procédé selon l'une quelconque des revendications 1 à 8, caractérisé par le fait que le catalyseur de déshydrogénation est déposé sur un support solide inerte présentant une surface spécifique supérieure à 50 $m^2$ par gramme.

**10.** Procédé selon l'une quelconque des revendications 1 à 9, caractérisé par le fait que l'acide glyoxylique et la cyclohexanone sont dissous dans de l'acide acétique ou dans un mélange eau-acide acétique.

**Patentansprüche**

**1.** Verfahren zur Herstellung von 2-Cumaranon, dadurch gekennzeichnet, dass man Glyoxylsäure mit Cyclohexanon in der Dampfphase in Gegenwart eines Dehydrierungskatalysators umsetzt.

**2.** Verfahren zur Herstellung von 2-Cumaranon, dadurch gekennzeichnet, dass man die durch eine nach einem an sich bekannten Verfahren durchgeführte Kondensation erhaltenen Reaktionsrohprodukte von Glyoxylsäure mit Cyclohexanon im Dampfzustand über einen Dehydrierungskatalysator leitet.

**3.** Verfahren zur Herstellung von 2-Cumaranon, dadurch gekennzeichnet, dass man eine Lösung von 2-Cyclohexanonessigsäure und/oder 2-Cyclohexanolessigsäure im Dampfzustand über einen Dehydrierungskatalysator leitet.

**4.** Verfahren nach Anspruch 1 oder 2, dadurch gekennzeichnet, dass es einen Recyclierungsschritt für die Reaktionsnebenprodukte von 2-Cyclohexanolessigsäure und 2-Cyclohexanonessigsäure nach dem Verfahren von Anspruch 3 umfaßt.

**5.** Verfahren nach irgendeinem der Ansprüche 1 bis 4, dadurch gekennzeichnet, dass man ein Trägergas einsetzt, aufgebaut aus einer Mischung von 100 bis 0% Stickstoff und 0 bis 100% Wasserstoff, um die Reaktionsmischung über den Dehydrierungskatalysator zu führen.

**6.** Verfahren nach irgendeinem der Ansprüche 1 bis 5, dadurch gekennzeichnet, dass man bei einer Temperatur über oder gleich 150°C arbeitet.

**7.** Verfahren nach irgendeinem der Ansprüche 1 bis 6, dadurch gekennzeichnet, dass der Dehydrierungskatalysator ausgewählt ist aus der Gruppe, bestehend aus Paladium und Platin.

**8.** Verfahren nach irgendeinem der Ansprüche 1 bis 7, dadurch gekennzeichnet, dass der Dehydrierungskatalysator auf einem festen inerten Träger abgeschieden ist, der eine spezifische Oberfläche von mehr als 1 $m^2$/g aufweist.

**9.** Verfahren nach irgendeinem der Ansprüche 1 bis 8, dadurch gekennzeichnet, dass der Dehydrierungskatalysator auf einem festen inerten Träger abgeschieden ist, der eine spezifische Oberfläche von mehr als 50 $m^2$/g aufweist.

**10.** Verfahren nach irgendeinem der Ansprüche 1 bis 9, dadurch gekennzeichnet, dass Glyoxylsäure und Cyclohexanon in Essigsäure oder einer Mischung Wasser/Essigsäure gelöst werden.

**Claims**

1. Preparation process for 2-coumaranone characterized in that glyoxylic acid is reacted in vapour phase with cyclohexanone, in the presence of a dehydrogenation catalyst.

2. Preparation process for 2-coumaranone characterized in that the crude reaction product obtained by condensation, carried out according to a process known per se, of glyoxylic acid with cyclohexanone is passed, in the vapour state, over a dehydrogenation catalyst.

3. Preparation process for 2-coumaranone, characterized in that a solution of 2-cyclohexanone acetic acid and/or 2-cyclohexanol acetic acid is passed, in the vapour state, over a dehydrogenation catalyst.

4. Process according to claim 1 or 2, characterized in that it contains a recycling stage of the by-products of the reaction, 2-cyclohexanol acetic acid and 2-cyclohexanone acetic acid, according to the process of claim 3.

5. Process according to any one of claims 1 to 4, characterized in that a supporting gas is used constituted by a mixture of 100 to 0% of dinitrogen and 0 to 100% of dihydrogen in order to entrain the reaction mixture over the dehydrogenation catalyst.

6. Process according to any one of claims 1 to 5, characterized in that the operation is carried out at a temperature higher than or equal to 150°C.

7. Process according to any one of claims 1 to 6, characterized in that the dehydrogenation catalyst is chosen from the group constituted by palladium or platinum.

8. Process according to any one of claims 1 to 7, characterized in that the dehydrogenation catalyst is deposited on an inert solid support having a specific surface area greater than 1 $m^2$ per gram.

9. Process according to any one of claims 1 to 8, characterized in that the dehydrogenation catalyst is deposited on an inert solid support having a specific surface area greater than 50 $m^2$ per gram.

10. Process according to any one of claims 1 to 9, characterized in that glyoxylic acid and cyclohexanone are dissolved in acetic acid or in a water-acetic acid mixture.